# EUROPEAN PATENT APPLICATION

(11) **EP 1 106 114 A1**
(43) Date of publication of application: **13.06.2001**
(21) Application number: 99124535.8
(22) Date of filing: 09.12.1999
(51) Int. Cl.: A47C 21/06, A61L 15/22, C08K 5/00, C08L 101/00

(54) **Disposable, moisture vapour permeable, liquid impermeable mattress cover having an improved structure for increased stability**

(71) Applicant: THE PROCTER & GAMBLE COMPANY, Cincinnati, Ohio 45202 (US)
(72) Inventor: Corzani, Italo, 66100 Chieti (IT); Russo, Elisabetta, 00142 Rom (IT)
(74) Representative: Veronese, Pancrazio

(57) **Abstract**

The present invention relates to a disposable mattress cover which is moisture vapour permeable and liquid impermeable and comprises an improved liquid impermeable formed structure having an enhanced moisture vapour permeability and comprising preferred thermoplastic compositions which can provide a better stability of the mattress cover in use. The disposable mattress cover of the present invention can find a variety of applications wherein moisture vapour permeability combined with liquid imperviousness are desirable, combined with stability in use.

## Description

### Field of the Invention

The present invention relates to disposable mattress covers which are moisture vapour permeable and liquid impermeable and comprise improved liquid impermeable formed structures having an enhanced moisture vapour permeability and comprising thermoplastic compositions preferably applied onto a moisture vapour permeable substrate. The mattress covers of the present invention can find a variety of applications wherein moisture vapour permeability combined with liquid imperviousness are desirable.

### Background of the Invention

Mattress covers, particularly bed mattress covers, are known in the art. A primary use of these articles is to protect mattresses, particularly bed mattresses, but also pillows, cushions, duvets, upholstery, from contaminants, e.g. from dust, liquids, or bodily fluids, for example in environments where a mattress is intended to be used by different users, such as in hospitals, hotels, or rental houses. Known mattress covers are either provided as structures that completely encase a mattress, or as layers covering the portion of the external surface of the mattress that has to be protected, typically at least a portion of the surface of the mattress which is intended, in use, to go in direct or indirect contact with the user, corresponding to the upper surface in a bed mattress, a cushion or a pillow. Such mattress covers are provided so that a user can use them in order to provide a mattress with a protective covering, which accordingly can be a partial covering, e.g. substantially limited to an upper surface as explained above, or a total encasing of the mattress.

More recently mattress covers have been also proposed to act as an allergen barrier to control house mites that live in the dust, typically on and within mattresses where they can find favourable environmental conditions in terms of temperature and nourishment.

It has long been established that house mites, also known as dust mites, are a source of house dust allergens that not only cause allergies, but also adversely contribute to other pathologies, such as asthma. It has also been established that use of allergen control measures is effective in controlling these conditions. Allergen-proof encasing to contain mites to prevent allergen egress has long been used in mattresses, such as bed mattresses, pillows, cushions, duvets, upholstery. Mattress covers to be used as allergen barrier against dust mites must therefore completely encase the mattress in order to separate the user from dust mites and related allergens.

Mattress covers come in direct or indirect (i.e. through further intermediate layers, e.g. bed sheets, or pillowcases) contact with the human body, therefore it is important that such covers are moisture vapour permeable for comfort reasons, in addition to being liquid impermeable in order to provide the mattress with the desired protection against external agents. When used as a barrier against dust mites they must also have an allergen barrier capability.

It is also preferred that mattress covers are disposable, that is, they can be discarded after use with no need of washing or refurbishing them.

Various examples of disposable mattress covers which are at the same time liquid impermeable and breathable, i.e. moisture vapour permeable, are known in the art, such as for example the bed mattress covers described in the French patent application FR 2747899.

Disposable breathable mattress covers known in the art comprise different materials or structures which are capable of providing a liquid barrier, in addition to providing moisture vapour permeability, preferably air permeability, and also having preferred characteristics in terms of softness, thickness and strength. Such structures or materials can comprise a single layer, or multiple layers laminated together. An example are structures comprising thermoplastic microporous films, e.g. laminated to fibrous layers such as nonwoven layers.

Particularly preferred materials which are suitable for disposable mattress covers are hydrophilic continuous films, also known as "monolithic films", that do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower.

In our patent applications EP 98110597.6 entitled "Low viscosity thermoplastic compositions for moisture vapour permeable structures and the utilisation thereof in absorbent articles", and EP 98110596.8 entitled "Low viscosity thermoplastic compositions for structures with enhanced moisture vapour permeability and the utilisation thereof in absorbent articles", both applications filed on 9 June 1998, thermoplastic compositions are disclosed for making hydrophilic continuous moisture vapour permeable, liquid impermeable layers having preferred characteristics of moisture vapour permeability and liquid imperviousness. The disclosed preferred thermoplastic compositions are also readily processable so as to provide a coating having the desired thickness onto a substrate, so avoiding the need of complex traditional extrusion apparatuses. This is achieved by modifying the viscosity of the thermoplastic polymers by means of the inclusion in the composition of a suitable plasticiser or blend of plasticisers that lowers such viscosity. This allows to utilise with these preferred compositions typical process conditions known in the art for the direct coating of low viscosity hot melt compositions onto a substrate in order to form a moisture vapour permeable, liquid impervious film or layer.

Particularly preferred plasticisers are described in EP 98110596.8.

Disposable, moisture vapour permeable, liquid impermeable mattress covers comprising a moisture vapour permeable, liquid impermeable structure, which in turn comprises a layer of the thermoplastic compositions according to either EP 98110597.6 or EP 98110596.8, preferably applied onto a moisture vapour permeable substrate, e.g. a fibrous layer such as a nonwoven layer in order to form a composite structure, are particularly preferred since they provide better moisture vapour permeability combined with liquid imperviousness, and also take advantage of the increased ease of manufacture associated to the above mentioned compositions. These preferred disposable mattress covers can however be still improved in terms of their interaction with a mattress.

It is in fact preferred that an increased friction is established between the disposable mattress cover and the mattress surface during the use, when i.e. the mattress cover is typically subjected to a certain compression against the mattress itself, in order to minimise possible misplacements of the cover during its use, for example when the mattress is a bed mattress or a pillow, and the mattress cover can be moved or misplaced by the user's movement, e.g. during the sleep. This is particularly important when the mattress cover is intended for partial covering of the external surface of a mattress, for example of the upper surface of a bed mattress, e.g., in combination with traditional means for connecting the cover to the mattress, such as for example elastic bands or strands applied at the corners of a rectangular bed mattress cover, according to an embodiment known in the art.

Provision of an increased friction between the mattress cover and the mattress is however also useful in the context of a mattress cover intended for total encasing of a mattress. In both cases in fact this increased friction prevents or at least reduces relative movements between the mattress cover and the mattress during the use, therefore helping in keeping the mattress cover in its right position provided initially, e.g. by the user when applying the cover to the mattress.

Preferred disposable mattress covers such as those mentioned above which preferably comprise a layered structure comprising the preferred thermoplastic compositions according to either EP 98110597.6 or EP 98110596.8 applied onto a fibrous substrate, e.g. a nonwoven layer substrate, typically have the thermoplastic composition which constitutes the inner layer of the liquid impermeable, moisture vapour permeable structure comprised in, or constituting the mattress cover, which inner layer is intended to face the mattress, and therefore to come in contact with it. Consequently, in the preferred layered structure the moisture vapour permeable substrate onto which the thermoplastic composition is applied, typically a fibrous layer, e.g. a nonwoven, constitutes the outer layer intended to come in direct or indirect contact with a user, which is also advantageous for comfort reasons.

It has been surprisingly discovered that by suitably selecting the components of the preferred thermoplastic compositions comprised in the liquid impermeable, moisture vapour permeable structure comprised in the disposable mattress cover of the present invention, it is possible to confer to the layer formed from the thermoplastic composition a certain tackiness which provides an increased friction towards the materials of a mattress with which this layer is intended to come in contact in use.

### Summary of the Invention

A disposable, moisture vapour permeable, liquid impermeable mattress cover comprising a moisture vapour permeable, liquid impermeable structure. The structure comprises a layer of a thermoplastic composition which is intended, in use, to directly contact a mattress.

The thermoplastic composition comprises:
from 10% to 80%, preferably from 25% to 70%, by weight of the thermoplastic composition, of a thermoplastic polymer or mixture of polymers selected from the group consisting of polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, or mixtures thereof;
from 20% to 90%, preferably from 30% to 75%, by weight of the thermoplastic composition, of a plasticiser or blend of plasticisers selected from the group consisting of citric acid esters, tartaric acid esters, glycerol and its esters, adipates, sebacates, sorbitol, epoxidized vegetal oils, polymerised vegetal oils, polyols, phthalates, liquid polyesters, glycolates, p-toluene sulfonamide and derivatives, glycols and polyglycols, sorbitan esters, phosphates, monocarboxylic fatty acids (C₈-C₂₂) and their derivatives, and mixtures thereof;
from 2% to 60%, preferably from 5% to 50%, more preferably from 10% to 40%, by weight of the thermoplastic composition, of a tackifier resin or a blend of tackifier resins selected from the group consisting of rosin and rosin esters, terpene-phenolic resins, aromatic resins, and mixtures thereof.

### Detailed Description of the Invention

According to the present invention, a disposable, moisture vapour permeable, liquid impermeable mattress cover is provided which comprises moisture vapour permeable, liquid impermeable structures comprising a layer of a highly processable thermoplastic composition having an enhanced moisture vapour permeability and being intended, in use, to directly contact a mattress. Said layer of said thermoplastic composition is capable of providing an increased friction between the mattress cover and a mattress in the use conditions.

The term "mattress", as used therein, refers to a fabric case filled with resilient material, such as for example cotton, hair, feathers, foam rubber, or an arrangement of coil springs, and therefore comprises particularly bed mattresses, but also pillows, cushions, comforters, duvets, upholstered portions of beds (such as headboards), or of sofas or armchairs.

While in principle the disposable, moisture vapour permeable, liquid impermeable mattress covers of the present invention can be comprised solely of the highly processable thermoplastic compositions capable of providing the increased friction, it is however preferred that said thermoplastic compositions are utilised in a composite structure in combination with one or more other materials to create a layered moisture vapour permeable, liquid impermeable composite structure comprised in the mattress cover. The composite structure, for example, can involve two or more components of a same specific thermoplastic composition within the present invention, or different specific thermoplastic compositions of the present invention.

Alternatively, and preferably, the composite structure can involve at least one component, typically a layer, of the thermoplastic composition in combination with one or more other moisture vapour permeable materials, provided that the component of the thermoplastic composition is intended, in use, to face the mattress and hence to come in direct contact with it. Such moisture vapour permeable materials include, but are not limited to: fibres, fibrous batts, nonwovens, wovens, papers, micro-porous or porous membranes, films such as polymeric films, perforated or apertured films and papers, macroscopically expanded films, cloth, etc.

Said other components may be non-absorbent, absorbent, liquid-containing, etc.

Preferably the composite structures described above have a moisture vapour transfer rate of at least 100 g/m²·24h, more preferably at least 300 g/m²·24h, and most preferably at least 500 g/m²·24h.

According to a particularly preferred embodiment of the present invention the disposable, moisture vapour permeable, liquid impermeable mattress cover comprises a composite structure comprising a layer of the thermoplastic composition applied onto a moisture vapour permeable substrate, wherein said layer is intended, in use, to directly contact the mattress. The moisture vapour permeable substrate preferably comprises a fibrous layer, e.g. a nonwoven layer onto which the thermoplastic composition is applied by means of known means, e.g. preferably by hot melt coating.

A disposable, moisture vapour permeable, liquid impermeable mattress cover comprising a moisture vapour permeable, liquid impermeable structure in turn comprising a layer of the thermoplastic composition according to the present invention which is intended, in use, to directly contact a mattress, can have any shape and arrangement which is known in the art, and which is suitable for its intended use.

For example, if the mattress consists of a standard bed mattress, the mattress cover can be a substantially rectangular flat sheet with elastic strings at the corners, or alternatively with integrally elasticised corners, as described in FR 2747899. The rectangular sheet typically has suitable dimensions in order to completely cover at least the upper surface of the bed mattress, and preferably, as in the latter embodiment referred to above, also the side surfaces thereof, wherein the elastic strings or the elasticised corners provide for a suitable releasable connection, in a way known in the art, to the mattress. Alternative configurations where the mattress cover has a suitable shape and size in order to completely encase a mattress are also possible. This latter configuration is particularly preferred when the mattress cover, in addition to liquid imperviousness and moisture vapour permeability, has also to provide a barrier against dust mites and related allergens. A closure of the mattress cover providing a tight seal by means of known means, e.g. adhesive means, is also particularly preferred in this case in order to completely isolate the mattress within the mattress cover itself.

Any other suitable shape or arrangement for a mattress cover according to the present invention is also possible, depending on the particular mattress type, shape and dimensions, and on the intended use of the mattress cover.

Suitable thermoplastic compositions for forming a layer comprised in the moisture vapour permeable, liquid impermeable structures comprised in the disposable mattress covers according to the present invention are thermoplastic compositions for making hydrophilic continuous moisture vapour permeable, liquid impermeable layers or films ("monolithic films") having preferred characteristics of moisture vapour permeability and liquid imperviousness. The thermoplastic compositions according to the present invention comprise preferred thermoplastic polymers such as polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28 weight %, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, or mixtures thereof.

Particularly preferred thermoplastic polymers are thermoplastic poly-ether-amide block copolymers (e.g. Pebax™), thermoplastic poly-ether-ester-amide block copolymers, thermoplastic polyester block copolymers (e.g. Hytrel™), thermoplastic polyurethanes (e.g. Estane™), or mixtures thereof.

Such thermoplastic polymers or mixture of polymers can be typically highly viscous in the melted state at the process conditions that are typical of the known processes of film or layer formation, e.g. an extrusion process involving a high power screw extruder. For example they may have a viscosity higher than 5000 poise at a temperature of 20°C above the DSC (Differential Scanning Calorimetry) melting point, which is the temperature identified as that corresponding to the DSC peak, or corresponding to the highest DSC peak in case of a mixture of polymers showing more than one peak, and at a frequency of 1 rad/sec.

The viscosity of the preferred thermoplastic polymers or mixture of polymers is adjusted by including in the thermoplastic composition a suitable plasticiser, or blend of plasticisers, that is compatible with the thermoplastic polymers and that lowers the viscosity of the thermoplastic polymer or mixture of polymers in the melted state.

The thermoplastic compositions according to the present invention comprising the suitable plasticiser or blend of plasticisers have the following complex viscosities (η∗):
50 poise < η∗ < 4000 poise, preferably 100 poise < η* < 2000 poise, more preferably 100 poise < η* < 1000 poise, at a frequency of 1 rad/s at a temperature of 210°C or less and η* < 2000 pose, preferably η* < 1000 poise, more preferably η* < 500 poise, at a frequency of 1000 rad/s at a process temperature (T) of 210°C or less, wherein η* represents the complex viscosity of the thermoplastic polymeric composition. Preferably the temperature T is 200°C or less and more preferably 180°C or less and most preferably from 200°C to 50°C.

The thermoplastic compositions having the complex viscosity described allow for a film or layer to be coated onto a substrate using typical coating conditions and apparatuses known in the art for the coating of low viscosities hot melt compositions in a layer having a required thickness onto a substrate, while also keeping the advantageous characteristics of the preferred thermoplastic polymers in providing hydrophilic continuous moisture vapour permeable, liquid impermeable layers or films.

Thermoplastic compositions having such viscosities can also provide very thin films or layers.

Suitable plasticisers or blend of plasticisers for adjusting such viscosity can be selected from the group consisting of citric acid esters, tartaric acid esters, glycerol and its esters, adipates, sebacates, sorbitol, epoxidized vegetal oils, polymerised vegetal oils, polyols, phthalates, liquid polyesters, glycolates, p-toluene sulfonamide and derivatives, glycols and polyglycols, sorbitan esters, phosphates, monocarboxylic fatty acids (C₈-C₂₂) and their derivatives, and mixtures thereof. This allows to utilise with these preferred compositions typical process conditions known in the art for the direct coating of low viscosity hot melts onto a substrate in order to form a moisture vapour permeable, liquid impervious film or layer.

It has also been found that by further selecting the plasticiser or blend of plasticisers to be comprised in the thermoplastic composition according to the present invention from the group of hydrophilic plasticisers consisting of acids, esters, amides, alcohols, polyalcohols, or mixtures thereof, the advantage of an enhanced moisture vapour permeability of the resulting layer or film formed from the thermoplastic composition is also achieved, when compared to a corresponding film or layer formed from a thermoplastic composition comprising the same thermoplastic polymer, but without the plasticiser.

The preferred hydrophilic plasticiser or blend of hydrophilic plasticisers can of course also adjust the viscosity of the thermoplastic composition according to the present invention to the preferred values in order to make it processable by coating said thermoplastic composition onto a substrate in a layer or film having a desired thickness.

Suitable preferred hydrophilic plasticisers according to this preferred embodiment of the present invention are citric acid esters, tartaric acid esters, glycerol and its esters, sorbitol, glycolates, and mixtures thereof.

The thermoplastic compositions comprised in the moisture vapour permeable, liquid impermeable mattress cover of the present invention also comprise a tackifier resin or a blend of tackifier resins. According to the present invention, it has been found that by suitably selecting the tackifier resin, or blend of tackifier resins, to be added to the thermoplastic composition, it is possible to adjust the residual tackiness of said thermoplastic composition at room temperature, i.e. in the solidified thermoplastic composition constituting the layer of the moisture vapour permeable, liquid impermeable structure comprised in the mattress cover, which layer is intended to directly contact the mattress in use. This in turn allows an adjustment of the friction that is established, in the use conditions, between the layer of said thermoplastic composition comprised in the disposable mattress cover of the present invention, and the mattress surface, when the mattress cover is typically subjected to compression against the mattress itself. An increased friction provides in fact a better stability of the liquid impermeable, moisture vapour permeable mattress cover of the present invention with respect to the mattress, avoiding or at least reducing the relative movements between mattress cover and mattress which can be induced in use, for example by the user's movements, e.g. during the sleep, when the mattress is a bed mattress or a pillow, and the mattress cover is a bed mattress or a pillow cover. Risk of misplacement of the mattress cover during the use is therefore greatly reduced. Moreover, this in turn allows the use of lighter and cheaper materials for the disposable mattress cover of the present invention, which is beneficial in the context of a disposable mattress cover. Since said materials comprised in the moisture vapour permeable, liquid impermeable structure comprised in the mattress cover of the present invention need to resist to a lesser stress during the use, they can be thinner, and therefore provide a better moisture vapour permeability in addition to liquid imperviousness, while being also softer and more comfortable for the user. The disposal of the mattress cover after use also implies a lesser waste of materials.

By suitably selecting the tackifier resin or blend of tackifier resins according to the present invention it is also possible to adjust the residual tackiness at room temperature of the thermoplastic composition, and therefore of the layer formed from said thermoplastic composition, to the extent that it has the typical characteristics of a pressure sensitive adhesive. In other words, the friction between the layer of thermoplastic composition comprised in the mattress cover of the present invention and the mattress can be increased such that the mattress cover can actually stick to the mattress, owing to the thermoplastic composition which can be formulated in order to behave like a pressure sensitive adhesive. Residual tackiness of the thermoplastic composition at room temperature can be suitably tailored according to the present invention since a smooth transition exists between increasing friction and actual adhesiveness.

Preferred tackifier resins, or blend of tackifier resins according to the present invention have a softening point of 125°C or less, and are selected from the group consisting of rosin and rosin esters, terpene-phenolic resins, aromatic resins, and mixtures thereof.

More preferably, the thermoplastic compositions according to the present invention comprise a blend of tackifier resins, selected as described above, wherein moreover from 0% to 20%, preferably from 2% to 15%, by weight of said blend of tackifier resins comprises a resin or blend of resins having a softening point of less than 25°C, and from 80% to 100%, preferably from 85% to 98%, by weight of said blend of tackifier resins comprises a resin or blend of resins having a softening point of at least 70°C.

The presence in the blend of tackifier resins of a certain amount of one or more tackifier resins which are liquid at room temperature is preferred since said resin or resins, in addition to adjusting the residual tackiness of the layer formed from the resulting thermoplastic composition, also contribute to lower the viscosity of the composition itself at the process conditions, in combination with the selected suitable plasticiser or blend of plasticisers.

Said preferred blend of tackifier resins therefore provides a thermoplastic composition for forming a layer in the moisture vapour permeable, liquid impermeable structure comprised in the mattress cover of the present invention, which is easily processable. Moreover the preferred blend of tackifier resins allows to adjust the residual tackiness of the resulting thermoplastic layer made from the thermoplastic composition, and therefore the friction between the mattress cover and the mattress in use conditions, which is beneficial as explained above.

The thermoplastic compositions comprised in the disposable, liquid impervious, moisture vapour permeable mattress covers according to the present invention comprise from 10% to 80%, preferably from 25% to 70% by weight of the thermoplastic composition, of the thermoplastic polymer or mixture of polymers, from 20% to 90%, preferably from 30% to 75% by weight of the thermoplastic composition, of a plasticiser or blend of plasticisers, and from 2% to 60%, preferably from 5% to 50%, more preferably from 10% to 40%, by weight of said thermoplastic composition, of a tackifier resin or blend of tackifier resins, said thermoplastic polymers, plasticisers, and tackifier resins selected as described above.

The thermoplastic compositions according to the present invention may in addition comprise additional optional components to further improve the processibility of the compositions and also the mechanical characteristics as well as other characteristics such as resistance to ageing by light and oxygen, visual appearance etc., of the films or layers formed from such thermoplastic compositions.

Such optional components include anti-oxidants, anti-ultraviolets, pigments and mixtures thereof, which may be present within the composition at a level of up to 10% by weight of the composition.

A thermoplastic composition according to the present invention can be manufactured with a process that will typically comprise the steps of providing the thermoplastic polymer or mixture of polymers, the suitable plasticiser or blend of plasticisers, and the tackifier resin or blend of tackifier resins, heating the components and compounding them, e.g. with a known suitable mixer to form the thermoplastic composition in the molten state having the desired complex viscosity η*.

According to the present invention a moisture vapour permeable, liquid impervious layer can be formed from the thermoplastic composition of the present invention by coating said thermoplastic composition onto a substrate. The films or layers formed from the thermoplastic compositions of the present invention preferably have a moisture vapour transport rate of at least 100 g/m²·24h, preferably at least 300 g/m²·24h, most preferably at least 500 g/m²·24h with a thickness of the layer or film of at least 0.5 µm.

A process for making a layer or film from a thermoplastic composition according to the present invention typically comprises the steps of providing said composition, heating it to make it flowable, and coating said composition in the molten state onto a substrate in a layer having the desired thickness. While said substrate can be simply a formation substrate, onto which the thermoplastic composition is coated in order to form a film or layer of the desired thickness which is subsequently separated from said substrate and used as such, in a preferred embodiment of the present invention a moisture vapour permeable, water impervious composite is formed which comprises the thermoplastic composition and a substrate onto which said thermoplastic composition is coated, wherein the substrate is also moisture vapour permeable.

Such embodiment provides a moisture vapour permeable, liquid impervious composite structure wherein the contribution of the layer formed from the thermoplastic composition of the present invention to the performance of the composite material resides only in the provision of a liquid barrier and hence could be advantageously provided as thinly as possible. The remaining performance physical criterion being preferably provided by the provided substrate, that therefore preferably acts also as a support layer. Typical thicknesses of the layer of the thermoplastic composition applied onto a suitable substrate to form a composite structure according to the present invention range from 2 µm to 200 µm.

The substrate, or support layer may be any useful layer which is also moisture vapour permeable, preferably having a moisture vapour permeability of at least 100 g/m²·24h, more preferably at least 300 g/m²·24h, and most preferably at least 500 g/m²·24h.

Suitable substrates for use herein as support layers include two dimensional, planar micro and macro-porous films; macroscopically expanded films; formed apertured films; nonwoven and woven layers. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable two dimensional porous planar layers of the backsheet may be made of any material known in the art, but are preferably manufactured from commonly available polymeric materials. Suitable materials are for example Goretex™ or Sympatex™ type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term two dimensional planar layer refers to layers having a depth of less than 1 mm, preferably less than 0.5 mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3,929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4,637,819 and US 4,591,523.

Preferred support layers for use herein include woven and nonwoven layers, most preferably hydrophobic fibrous layers such as hydrophobic nonwoven.

The composites of this preferred embodiment according to the present invention are particularly advantageous as they allow the possibility of providing a composite wherein the thermoplastic composition may be coated onto the support substrate as a layer with the desired thickness. Typical coating conditions and apparatuses known in the art for the direct coating of low viscosities hot melts can be readily utilised in order to provide the thermoplastic composition at the desired thickness.

A possible method for forming a composite laminate by coating the thermoplastic composition onto a substrate acting as a support layer is described in PCT application WO 96/25902.

At least at the coating temperature, the thermoplastic composition in form of a layer preferably exhibits adhesive properties on the supportive substrate in order to form the preferred composite such that no additional adhesive is required to achieve a permanent attachment between the thermoplastic composition and the substrate.

Preferably the moisture vapour permeable, liquid impervious structures, e.g. layers or composites comprising the thermoplastic compositions according to the present invention, and comprised in the mattress covers of the present invention have an overall moisture vapour transfer rate of at least 100g/m²·24h, more preferably at least 300 g/m²·24h, and most preferably at least 500 g/m²·24h.

A moisture vapour permeable, liquid impervious composite structure formed by coating the thermoplastic composition of the present invention onto a suitable substrate finds particular utility as a structure constituting a disposable, moisture vapour permeable, liquid impermeable mattress cover according to the present invention.

### Example:

A polyether-amide block copolymer available from Elf Atochem (France) commercialised under the trade name Pebax 2533 SN is compounded with Tri Butyl Citrate (plasticiser) available from Aldrich Co., Foral 85-E (tackifier resin) available from Hercules Company, and Irganox 1010 (anti oxidant agent) available from Ciba-Geigy.

The final formulation in percent by weight has the following composition:

| | |
|---|---|
| 33% | Pebax 2533 SN |
| 33% | Tri Butyl Citrate |
| 33% | Foral 85-E |
| 1% | Irganox 1010 |

The blend is melt extruded to obtain a film having a thickness equal to 15µm. The film is laminated directly onto a substrate constituted by a carded hydrophobic 100% polypropylene nonwoven 34 g/m² (support layer) commercialised under the trade name Sawabond 4124, available form Sandler (Germany).

The composite structure is used to produce a mattress cover, e.g. a bed mattress cover having a flat rectangular shape with suitable dimensions to completely cover at least the upper surface of a bed mattress, wherein the layer of thermoplastic composition is intended to face, in use, the mattress, and to directly contact it.

According to the present invention the complex viscosity η* is measured using a Rheometer RDA-II available from Rheometrics Co. Moisture vapour permeability is measured as Water Vapour Transmission Rate (WVTR) at 23°C according to the ASTM E-96 "Upright Cup" method. The softening point of the resins is measured according to the Ring and Ball ASTM E28 method.

## Claims

1. A disposable, moisture vapour permeable, liquid impermeable mattress cover comprising a moisture vapour permeable, liquid impermeable structure, said structure comprising a layer of a thermoplastic composition which is intended, in use, to directly contact a mattress,
said thermoplastic composition comprising:
from 10% to 80%, preferably from 25% to 70%, by weight of said thermoplastic composition, of a thermoplastic polymer or mixture of polymers selected from the group consisting of polyurethanes, poly-ether-amides block copolymers, polyethylene-acrylic acid copolymers, polyethylene oxide and its copolymers, ethylene acrylic esters copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28% by weight, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, or mixtures thereof;
from 20% to 90%, preferably from 30% to 75%, by weight of said thermoplastic composition, of a plasticiser or blend of plasticisers selected from the group consisting of citric acid esters, tartaric acid esters, glycerol and its esters, adipates, sebacates, sorbitol, epoxidized vegetal oils, polymerised vegetal oils, polyols, phthalates, liquid polyesters, glycolates, p-toluene sulfonamide and derivatives, glycols and polyglycols, sorbitan esters, phosphates, monocarboxylic fatty acids (C₈-C₂₂) and their derivatives, and mixtures thereof;
from 2% to 60%, preferably from 5% to 50%, more preferably from 10% to 40%, by weight of said thermoplastic composition, of a tackifier resin or a blend of tackifier resins selected from the group consisting of rosin and rosin esters, terpene-phenolic resins, aromatic resins, and mixtures thereof.

2. A disposable, moisture vapour permeable, liquid impermeable mattress cover according to claim 1, wherein said structure comprises said layer of said thermoplastic composition applied onto a substrate, said substrate being moisture vapour permeable.

3. A disposable, moisture vapour permeable, liquid impermeable mattress cover according to any preceding claim, wherein said blend of tackifier resins comprises from 0% to 20%, preferably from 2% to 15%, by weight of said blend of tackifier resins, of a resin or blend of resins having a softening point of less than 25°C, and from 80% to 100%, preferably from 85% to 98%, by weight of said blend of tackifier resins, of a resin or blend of resins having a softening point of at least 70°C, said softening points measured according to the Ring and Ball ASTM E28 method.

4. A disposable, moisture vapour permeable, liquid impermeable mattress cover according to any preceding claim, wherein said layer of said thermoplastic composition is liquid impervious and has a water vapour transmission rate (WVTR) of at least 300 g/m²·24h with a thickness of said layer of at least 0.5 µm.
